(19) Europäisches Patentamt / European Patent Office / Office européen des brevets

(11) **EP 1 781 161 B1**

(12) **EUROPÄISCHE PATENTSCHRIFT**

(45) Veröffentlichungstag und Bekanntmachung des Hinweises auf die Patenterteilung:
**10.02.2010 Patentblatt 2010/06**

(21) Anmeldenummer: **05769744.3**

(22) Anmeldetag: **26.07.2005**

(51) Int Cl.:
*A61B 3/107* *(2006.01)*    *A61B 3/103* *(2006.01)*

(86) Internationale Anmeldenummer:
**PCT/EP2005/008090**

(87) Internationale Veröffentlichungsnummer:
**WO 2006/015717 (16.02.2006 Gazette 2006/07)**

(54) **FOURIER-DOMAIN OCT RAY-TRACING AM AUGE**

FOURIER-DOMAIN OCT RAY-TRACING ON THE EYE

LANCER DE RAYON OCT DANS LE DOMAINE FOURIER SUR UN OEIL

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.08.2004 DE 102004037479**

(43) Veröffentlichungstag der Anmeldung:
**09.05.2007 Patentblatt 2007/19**

(73) Patentinhaber: **Carl Zeiss Meditec AG**
**07745 Jena (DE)**

(72) Erfinder: **FERCHER, Adolf, Friedrich**
**A-1230 Wien (AT)**

(56) Entgegenhaltungen:

• **ZULUAGA A F ET AL: "Spatially resolved spectral interferometry for determination of subsurface structure" OPTICS LETTERS OPT. SOC. AMERICA USA, Bd. 24, Nr. 8, 15. April 1999 (1999-04-15), Seiten 519-521, XP002350079 ISSN: 0146-9592**

• **ZAWADZKI R J ET AL: "Three-dimensional ophthalmic optical coherence tomography with a refraction correction algorithm" PROCEEDINGS OF THE SPIE - THE INTERNATIONAL SOCIETY FOR OPTICAL ENGINEERING SPIE-INT. SOC. OPT. ENG USA, Bd. 5140, Nr. 1, 2003, Seiten 20-27, XP002350080 ISSN: 0277-786X**

• **WOJTKOWSKI M ET AL: "In vivo human retinal imaging by Fourier domain optical coherence tomography" JOURNAL OF BIOMEDICAL OPTICS SPIE USA, Bd. 7, Nr. 3, Juli 2002 (2002-07), Seiten 457-463, XP002272406 ISSN: 1083-3668**

• **CORONA E ET AL: "Digital stereo image analyzer for generating automated 3-D measures of optic disc deformation in glaucoma" IEEE TRANSACTIONS ON MEDICAL IMAGING IEEE USA, Bd. 21, Nr. 10, Oktober 2002 (2002-10), Seiten 1244-1253, XP002350082 ISSN: 0278-0062**

**Beschreibung**

Die vorliegende Erfindung betrifft ein Messverfahren für die Ophthalmologie, welches mittels Kurzkohärenz-Interferometrie die Positionen von Stützpunkten misst, auf deren Basis die dreidimensionale Struktur aller intraokulären brechenden und reflektierenden Grenzflächen und Oberflächen beispielsweise mittels Spline- oder Polygonflächen dargestellt werden kann.

Hierzu werden durch eine Anzahl von Pupillenpunkten simultan Messstrahlen in das Auge gestrahlt und die Tiefenpositionen der Durchstoßpunkte und Reflexionspunkte dieser Messstrahlen an intraokulären Grenzflächen und Oberflächen mittels spektralinterferometrischer Kurzkohärenz-Interferometrie (auch als Fourier Domain OCT bekannt) bestimmt. Auf der Basis dieser Durchstoßpunkte und Reflexionspunkte kann die Form dieser Flächen numerisch berechnet werden und zur dreidimensionalen Darstellung der Struktur des Auges mittels bekannter Computergraphik-Techniken, wie Spline-Flächen oder Polygonflächen benutzt werden.

Das Messobjekt wird dazu in den einen Arm eines Zweistrahl-Interferometers gestellt. Das Spektrum des vom Messobjekt kommenden Lichtbündels wird mit jenem Referenzbündel am Interferometerausgang überlagert und die resultierende spektrale Intensität spektrometrisch ausgewertet.

In den letzten etwa 15 Jahren wurden verschiedene experimentelle Ray-Tracing Methoden entwickelt, die zur Messung der Eigenschaften optischer Systeme geeignet sind. So beleuchten Navarro und Losada ("Aberrations and relative efficiency of light pencils in the living human eye", in Optometry-and-Vision-Science 74(7), 540-547, 1997) die Pupille des Auges mit parallelen Laserstrahlen und bestimmen die Abbildungseigenschaften des Auges aus der Position des remittierten Strahls auf der zur Retina abbildungsmäßig konjugierten Photokathode einer CCD Kamera. Die Anwendung dieses Verfahrens zur Messung der Abbildungseigenschaften von Optiken haben Navarro und Moreno-Barriuso ("Laser ray-tracing method for optical testing", in Optics Letters 24(14), 951-953, 1999) beschrieben. Bei diesem Ray Tracing Verfahren wird allerdings die kumulative Gesamtwirkung aller brechenden Flächen gemessen. Die Bestimmung der Form einzelner Grenzflächen und damit eine kausale Zuordnung von Abbildungsfehlern des Auges an die verursachenden Grenzflächen ist nur näherungsweise möglich.

Ray-Tracing ist heute hauptsächlich bekannt als rechnerisches Verfahren von Graphik-Programmen zur Darstellung realistischer Beleuchtungsverhältnisse beim dreidimensionalen Wiedergeben (Rendering) von Computer-Bildern. Ray-Tracing ist jedoch neben Wavefront-Tracing auch ein numerisches Verfahren zur Berechnung der Lichtausbreitung in inhomogenen Medien. Während das auf den Brechungsgesetzen beruhende Ray-Tracing bereits in der Optik zum Optikdesign benutzt wird, ist die Anwendung des auf dem Huygens-Prinzip beruhenden Wavefront-Tracing eher in der Seismologie zur Berechnung der Ausbreitung seismischer Wellen bekannt.

In der optischen Kohärenztomographie werden Bilder aus kurzkohärenzinterferometrischen Messdaten von sogenannten A-Scans erzeugt. Diese A-Scans bestimmen die Objektstruktur als Verteilung lichtremittierender Stellen entlang der Messstrahlen in der Objekttiefe. Dabei wird in dem Kurzkohärenz-Interferometer das vom Objekt remittierte Licht mit einem Referenzstrahl durch Verschieben des Referenzspiegels korreliert. Nach den bekannten Regeln der Fourier-Domain OCT hingegen werden die A-Scan Daten durch Fouriertransformation der Intensitätsspektren des vom Objekt remittierten und mit einem Referenzstrahl überlagerten Lichts berechnet (Bouma, B. E., Tearney, G. J.; "Handbook of Optical Coherence Tomography"; Marcel Dekker Verlag New York; Kapitel 12; 2002).

Für Zwecke des Ray-Tracing am Auge ist es erforderlich, die Pupille des Auges in mehreren verteilten Pupillenpunkten zu beleuchten und die Messstrahlen entsprechend auszuwerten. Hierzu ist in der bekannten Time-Domain OCT ein Pupillenscan erforderlich, bei dem der Messstrahl konsekutiv mittels einer Scanningeinheit in die verschiedenen Pupillenpunkte gelenkt wird. In jedem Punkt wäre das Intensitätsspektrum des A-Scan zu messen. Das benötigt viel Zeit und kann zu Bewegungsartefakten führen.

Als kurzkohärente Beleuchtungsquelle kann man hierfür beispielsweise eine Superlumineszenzdiode, eine lichtemittierende Diode (LED), einen Modelock Laser, eine ASE (= Amplified Spontanous Emission) Faserlichtquelle, eine Photonic Crystal Faser Lichtquelle, eine thermische Lichtquelle (Glühlampe) oder eine Plasmalichtquelle (Bogenlampe) verwenden.

Im folgenden wird zunächst auf ähnliche Fourier-optische OCT Verfahren eingegangen, die nach dem Stand der Technik bekannt sind.

In Publikationen von A. F. Fercher und Mitarbeitern ("Measurement of optical distances by optical spectrum modulation", Proc. SPIE Vol. 2083, 263-267, 1993) wurden das Fourier-optische OCT-Verfahren im allgemeinen und auch die spezielle Bestimmung der Kohärenzfunktion des vom Auge reflektierten Lichtes durch inverse Fourier-Transformation der spektralen Intensitätsverteilung I(□) beschrieben ("In Vivo Optical Coherence Tomography in Ophthalmology", Bellingham, W. A.: SPIE. pp. 355-370, ISBN 0-8194-1379-8, 1993).

Die Verwendung des Fouriertransformations-Verfahrens speziell zur Messung intraokulärer Distanzen entlang einem Einzelstrahl durch die Pupille wurde von A. F. Fercher und Mitarbeitern ("Measurement of Intraocular Distances by Backscattering Spectral Interferometry", Opt. Commun. 117, 43-48, 1995) beschrieben und durch G. Häusler und M. W. Lindner zur Herstellung von OCT-Bildern benutzt ("Coherence RADAR" and "spectral RADAR"-New tools for dermatological diagnosis, J. Biomed. Opt. 3(1), 21-31, 1998).

In der DE 43 09 056 A1 wird ein Verfahren zur Ermittlung der Entfernung und Streuintensität von streuenden Punkten beschrieben, bei dem die Entfernung und die lokale Streuintensität durch Fouriertransformation des Spektrums nach der Wellenlänge bestimmt wird.

Ein Verfahren bei dem dreidimensionale Bilder der Retina aus En-face OCT Aufnahmen synthetisiert werden können, wurde von A. G. Podoleanu, J. A. Rogers, D. A. Jackson, und S. Dunne beschrieben ("Three dimensional OCT images from retina and skin", Opt. Express, 7, pp. 292-298, 2000).

Ein Parallel-OCT Verfahren, welches ebenfalls einen Stufen-Referenzspiegel nutzt, ist in der US 6,268,921 B1 beschrieben. Der Stufen-Referenzspiegel wird hierbei zur Realisierung des Tiefen-Scans bei der sogenannten Time-Domain OCT verwendet. Dementsprechend sind die Stufengrößen auch deutlich größer als □/8. Die Stufen sind außerdem nicht mit periodisch wiederkehrenden Gesamthöhen sondern über die gesamte Fläche treppenförmig verteilt. Der in dieser Lösung weiterhin verwendete Phasenshifter wirkt auf den gesamten Referenz- oder Messarm gleich. Diese Unterschiede ergeben sich natürlich aus der dort auch vorliegenden anderen Problemstellung.

Ein ähnliches, auf piezoelektrischem Phase-Shifting Phasenmessung beruhendes Verfahren, ist Inhalt der US 6,377,349 B1. Bei dieser Lösung wird der Referenzspiegel piezoelektrisch verschoben. Diese Verschiebung und die erforderlichen zusätzlichen Belichtungen und das mehrfache Auslesen des Photodetektor-Arrays benötigen jedoch Zeit, was bei lebenden Objekten wie dem Auge zu Bewegungsartefakten führt.

Ein konventionelles OCT-Verfahren zur Bestimmung der Abmessungen der vorderen Augenabschnitte, unter Verwendung einer Spaltlampe und eines Handgerätes, wurde von S. Radhakrishnan und anderen ("Real time optical coherence tomography of the anterior segment using hand-held and slit-lamp adapted systems", Proc. SPIE 4619, 227-229, 2002) beschrieben. Das auf Time-Domain OCT beruhende Gerät arbeitet sehr schnell und liefert acht Bilder je Sekunde. Beispielsweise kann man für eine dreidimensionale Darstellung der vorderen Augenstruktur die 8 Bilder je Sekunde auf die gesamte Pupille äquidistant verteilen; dann benötigt man etwa 1 Sekunde zur Datenaufnahme. Demgegenüber kann die der vorliegenden Anmeldung zugrunde liegende Methode die erforderlichen Daten in Millisekunden registrieren.

Ein weiteres Verfahren ist von Zuluaga et al., "Spatially resolved spectral interferometry for determination of subsurface structure", optics Letters vol. 24, no.8 bekannt.

Der vorliegenden Erfindung liegt daher die Aufgabe zugrunde, Stützpunkten für die dreidimensionale Darstellung der Struktur der intraokulären brechenden und reflektierenden Grenzflächen und Oberflächen mittels Spline- oder Polygonflächen sehr schnell zu messen.

Die erfindungsgemäße Lösung ist in Anspruch 1 definiert.

Entsprechend der Erfindung erfolgt dies mittels Fourier-Domain Distanzmessung simultan in vielen über die Augenpupille verteilten Messstrahlen.

Bei dem erfindungsgemäßen Fourier-Domain Ray-Tracing am Auge wird die Pupille von einer kurzkohärenten Beleuchtungsquelle in mehreren Punkten mit in das Auge eindringenden Messstrahlen beleuchtet und die in den Durchstoßpunkten der Messstrahlen von Grenzflächen und Oberflächen des Auges reflektierte Messstrahlung wird mit einer Referenzstrahlung überlagert. Die mit der Referenzstrahlung überlagerte Messstrahlung wird am Ausgang des Interferometers beispielsweise durch ein Beugungsgitter spektral aufgespalten und auf ein zweidimensionales Detektorarray abgebildet. Die resultierenden elektrischen Signale werden an eine Steuereinheit weitergeleitet. Diese Steuereinheit ermittelt mittels Fourier-Transformation die Position der erwähnten Durchstoßpunkte entlang den Messstrahlen. Diese Durchstoßpunkte bilden die Stützpunkte für die Darstellung der dreidimensionalen Struktur aller intraokulären brechenden und reflektierenden Grenzflächen und Oberflächen des Auges.

Die Erfindung wird nachfolgend anhand von Ausführungsbeispielen näher beschrieben. Die Erklärung erfolgt anhand eines Michelson-Interferometers, obwohl auch andere Typen verwendbar sind. Dazu zeigen

Figur 1:    ein erfindungsgemäßes Kurzkohärenz-Interferometer mit divergent auf die Pupille auftreffenden Messstrahlen,

Figur 2:    ein erfindungsgemäßes Kurzkohärenz-Interferometer mit parallel auf die Pupille auftreffenden Messstrahlen,

Figur 3:    das Bild der Iris 74 mit Pupillenpunkten 73,

Figur 4:    ein erfindungsgemäßes Kurzkohärenz-Interferometer, mit einem um den Winkel β verdrehten Beugungsgitter 16,

Figur 5:    eine Illustration des durch Drehung des Beugungsgitters 16 erzielbaren Gewinns an spektraler Auflösung.

Figur 6:    ein erfindungsgemäßes Phasenmultiplex-Kurzkohärenz-Interferometer,

Figur 7:    ein erfindungsgemäßes Phasenmultiplex-Kurzkohärenz-Interferometer, bei welchem das Beugungsgitter um den Winkel β verdreht ist,

Figur 8:    die Anordnung von Schlitzblenden auf einer rotierenden Scheibe und

Figur 9: ein erfindungsgemäßes Phasenmultiplex-Kurzkohärenz-Interferometer mit einem Linsenraster im Beleuchtungsstrahl des Interferometers.

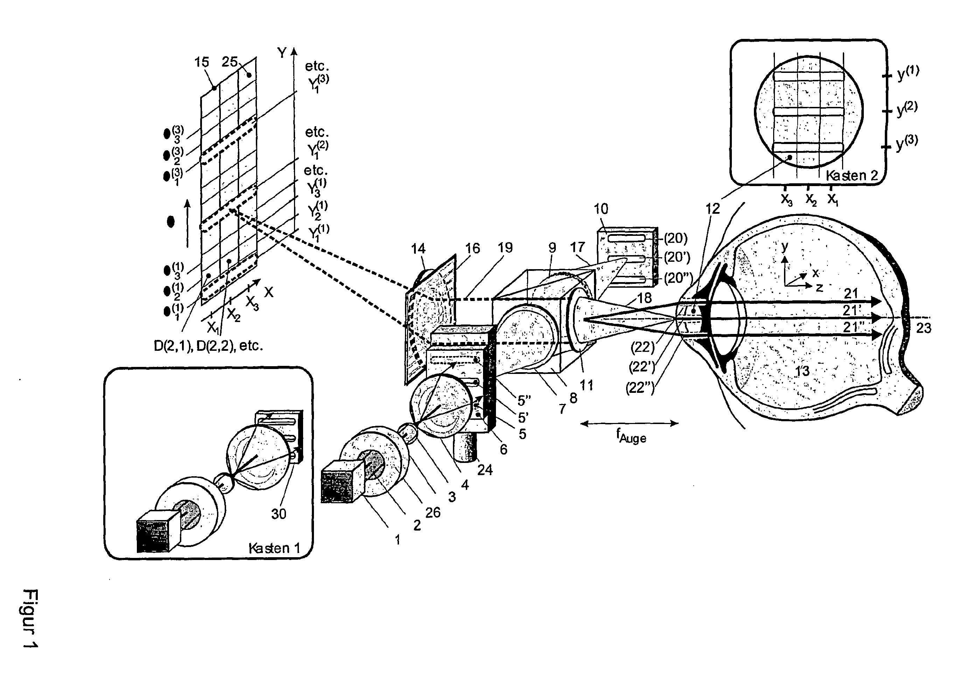

**Figur 1** zeigt ein erfindungsgemäßes Kurzkohärenz-Interferometer für Ray-Tracing, mit divergenter Beleuchtung des Auges. Hier beleuchtet der von der kurzkohärenten Beleuchtungsquelle **1** ausgehende und durch den Verschluss **26** hindurch tretende Lichtstrahl **2** mittels der Optiken **3** und **4** eine in y-Richtung von einem Antrieb **24** bewegte Lichtschlitzöffnung **5**. Die Lichtschlitzblende **6** kann dabei statt eines Schlitzes auch über eine Reihe von Öffnungen verfügen. Der Lichtschlitz der Lichtschlitzblende **6** wird konsekutiv in die Positionen **5, 5'** und **5"** bewegt. Die Optik **7** bildet die Lichtschlitzöffnung einerseits durch den Strahlteiler **8** hindurch mit der Optik **9** auf den Referenzspiegel **10** und andererseits über die Teilerfläche des Strahlteilers **8** mit der Optik **11** auf die Pupille **12** des Auges **13** ab. Die den unterschiedlichen Positionen **5, 5'** und **5"** der Lichtschlitzblende **6** entsprechenden Lichtschlitzbilder **20, 20'** und **20"** auf dem Referenzspiegel **10** bzw. **22, 22'** und **22"** in der Pupille **12** des Auges **13** werden von den Optiken **11, 9** und **14** auf das Detektorarray **15** in Positionen $D_j(1)$, $Y_j(2)$ und $Y_j(3)$ mit j = 1, 2, 3, ... abgebildet.

Die Optik **14,** das Detektorarray **15** und das Beugungsgitter **16** bilden ein Spektrometer, welches das Intensitätsspektrum des aus dem Kurzkohärenz-Interferometer nach dem Strahlteiler **8** in Richtung Detektorarray **15** austretenden Lichtstrahls **19** spektral analysiert. Der Lichtstrahl **19** besteht aus den einander überlagernden reflektierten Objektstrahlen **18** und Referenzstrahlen **17.**

Die Detektoren **25** des Detektorarrays **15** sind in Spalten mit gleichen X-Werten $X_1$, $X_2$ usw. und in Zeilen mit gleichen Y-Werten $Y_j^{(k)}$ angeordnet, wobei j der Wellenlängen-Nummer und k der Lichtschlitznummer entspricht.

Bei der Datenaufnahme wird der Lichtschlitz der Lichtschlitzblende **6,** angetrieben durch einen Motor **24,** in y-Richtung konsekutiv in die Positionen **5, 5', 5"** usw. verschoben, wodurch die Pupille **12** von den Lichtschlitzbildern **22, 22'** und **22"** und der Referenzspiegel **10** von den Lichtschlitzbildern **20, 20'** und **20"** in entsprechenden y-Positionen $y^{(1)}$, $y^{(2)}$ und $y^{(3)}$ konsekutiv beleuchtet wird.

Die durch Überlagerung der Referenzstrahlen **17** und der Objektstrahlen **18** entstehenden Lichtstrahlen **19** erzeugen auf dem Detektorarray **15** in den Positionen $Y_j^{(1)}$, $Y_j^{(2)}$ und $Y_j^{(3)}$ Lichtschlitzbilder mit überlagerter Referenzstrahlung. Zwischen den Positionen $Y_1^{(1)}$, $Y_1^{(2)}$ und $Y_1^{(3)}$ liegen auf dem Detektorarray **15** Detektorzeilen auf die das Spaltspektrum dispergiert wird. Den Positionen $(X_j; Y_j^{(k)}$ auf dem Detektorarray **15** entsprechen die Positionen $(x_i; y^{(k)})$ auf der Pupille 12; mit "j" sind die spektralen Komponenten gekennzeichnet.

Die Dispersion des Beugungsgitters **16** verteilt die spektralen Komponenten des Lichtstrahls **19** aus Positionen $y^{(k)}$ auf die Wellenlängen $\lambda_1^{(k)}$ $\lambda_2^{(k)}$ usw.. Wie im Kasten 2 in **Figur 1** veranschaulicht, gehört zu einem Punkt $(x_i; y^{(k)})$ in der Pupille **12,** die spektrale Intensität $I_i^{(k)}(\lambda_j)$ in der Position $(X_i; \lambda_j^{(k)})$ für j = 1, 2, 3, ... am Detektorarray **15**. Die hier von den Detektoren **25** in der Spalte (für jeweils konstantes i) $X_i$ und den Zeilen $V_j^{(k)}$ für k = 1, 2, ... in Abhängigkeit von der Wellenlänge $(\lambda_j^{(k)})$ gemessenen spektralen Intensitäten $I_i^{(k)}(\lambda_j)$ bilden die Daten für die Fouriertransformation zur Berechnung der Objektstruktur nach den Regeln der Fourier Domain OCT. Die wellenlängenabhängigen Messdaten müssen hierzu auf Wellenzahl-Abhängigkeit umgerechnet werden. Durch die Fourier-Transformation dieser Daten werden dann die z-Koordinaten der Stützpunkte entlang einem Messstrahl in den abbildungsmäßig konjugierten Pupillenpositionen $x_i$, $y^{(k)}$ am Auge **13** bestimmt.

Das zur Dispersion des Lichtstrahls **19** erforderliche Beugungsgitter **16** kann auch als Reflexionsgitter oder als Dispersionsprisma ausgeführt sein.

Es gibt hier zwei grundsätzliche Modi, das Spektrum auszulesen. In beiden Modi wird der Verschluss **26** immer nur geöffnet, wenn sich der Lichtschlitz in den Positionen **5, 5', 5"** usw. befindet. Beim simultanen Modus werden die Spektren aller Lichtschlitzpositionen elektronisch gleichzeitig ausgelesen. Dazu werden die aus den verschiedenen Schlitzpositionen anfallenden Intensitätswerte zunächst am Array abgespeichert. Für die spektrale Auflösung und damit für die Tiefenauflösung stehen die zwischen den $Y_1^{(k)}$-Positionen liegenden Detektorzeilen zur Verfügung. Dieser Modus kann wegen des simultanen Auslesens des gesamten Spektrums sehr schnell sein. Allerdings ist die Feldtiefe durch die begrenzte Anzahl der zwischen den $Y_1^{(k)}$-Positionen liegenden Detektorzeilen limitiert.

Beim konsekutiven Modus hingegen werden die Spektren sofort ausgelesen, wenn sich der Lichtschlitz in den Positionen **5, 5', 5"** usw. befindet. Dieser Modus ist jedoch langsamer als der simultane, weil der hier viel häufiger erforderliche Ausleseprozess verzögernd wirkt. Allerdings hat der konsekutive Modus den Vorteil, dass für das λ-Spektrum deutlich mehr Detektorzeilen (z. B. alle oberhalb der Position $Y_1^{(i)}$) zur Verfügung stehen. Dadurch erhöht sich die Feldtiefe.

Es sei darauf hingewiesen, dass das erfindungsgemäße Verfahren hier nur für 3 Spalten $(X_1, X_2$ und $X_3)$ sowie 3 Zeilen $(Y_1^{(1)}, Y_1^{(2)}$ und $Y_1^{(3)})$ beschrieben wird. Das Verfahren kann für beliebige Spalten- und Zeilenzahlen realisiert werden, wobei diese Zahlen durch die Spalten- und Zeilenzahlen des Detektorarrays **15** begrenzt werden.

Neben Zeitverzögerungen durch den Ausleseprozess des Detektorarrays **15** benötigt auch die Scan-Bewegung des Lichtschlitzes Zeit. Dieser Zeitverzug lässt sich durch einen Lichtschlitzraster vermeiden. Wie im Kasten 1 in **Figur 1** dargestellt, beleuchtet der aus der kurzkohärenten Beleuchtungsquelle **1** ausgehende Lichtstrahl **2** mittels der Optiken **3** und **4** nun einen Lichtschlitzraster **30.**

Die weitere Abbildung des Lichtschlitzrasters **30** erfolgt entsprechend der bereits beschriebenen Lösung der von einem Motor **24** bewegten Lichtschlitzblende **6** mit nur einem Lichtschlitz. Auch hier entsprechen den unterschiedlichen Positionen der Lichtschlitze des Lichtschlitzrasters **30** die Lichtschlitzbilder **20, 20'** und **20"** auf dem Referenzspiegel **10, 22, 22'** und **22"** in der Pupille **12** des Auges **13** bzw. $Y^{(1)}$, $Y^{(2)}$ und $Y^{(3)}$ auf dem Detektorarray **15.**

Im dargestellten Strahlengang nach **Figur 1** befindet sich die Optik **11** in der vorderen Brennebene des Auges **13.** Die Pupille **12** wird dadurch mit divergenten Messstrahlen **21, 21'** und **21"** beleuchtet, deren Divergenzzentrum im vorderen Brennpunkt des Auges **13** liegt. Wie in **Figur 1** angedeutet, werden diese Messstrahlen von der Optik (Cornea und Augenlinse) des Auges **13** parallel gerichtet.

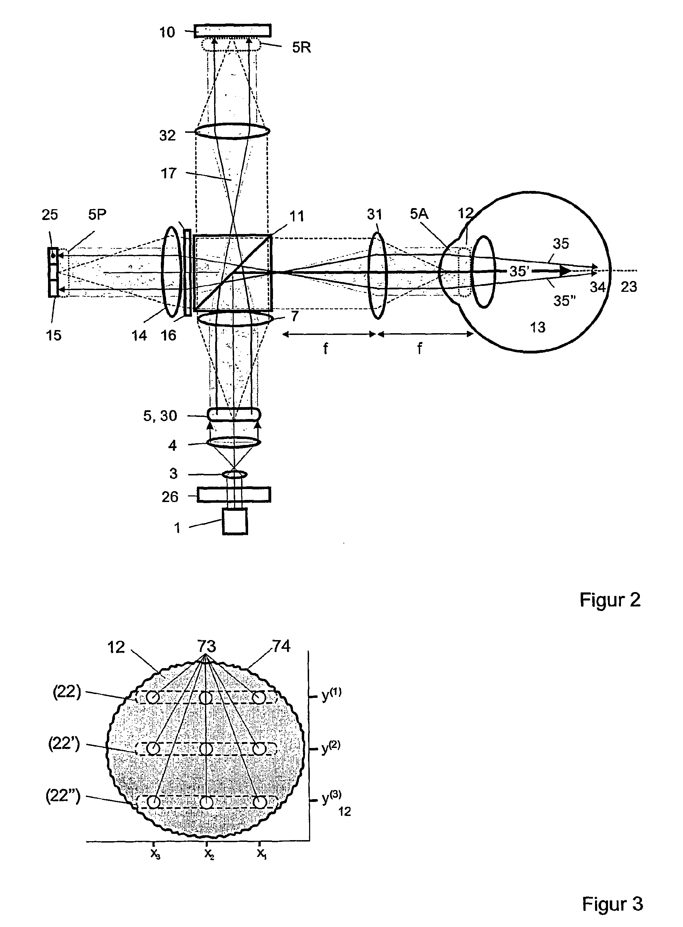

Alternativ dazu kann die Pupille **12** des Auges **13** mit parallelen Messstrahlen beleuchtet werden, wie in der **Figur 2** dargestellt. Bei paralleler Beleuchtung wird der Lichtschlitz der Lichtschlitzblende **6** bzw. der Lichtschlitzraster **30** von der Optik **7** zunächst nach Unendlich abgebildet und hinter dem Strahlteiler **11** von der Optik **32** auf den Referenzspiegel **10** und von der Optik **31** auf die Pupille **12** fokussiert.

Der wichtigste Unterschied beider Beleuchtungsvarianten liegt im Strahlenverlauf innerhalb des Auges **13.** Während die Messstrahlen **21, 21'** und **21"** bei divergenter Beleuchtung gemäß **Figur 1** ein etwa zylinderförmiges Volumen erfassen können, verlaufen die Messstrahlen (**35, 35'** und **35"**) bei paralleler Beleuchtung im Auge **13** konvergent. Von dem (entspannten) Auge **13** werden diese entsprechend den normalen Sehstrahlen auf dem Augenfundus fokussiert.

Die bisher beschriebenen Anordnungen leiden darunter, dass bei einer größeren Anzahl von Messstrahlen der Tiefenmessbereich wegen der dann geringeren Pixelzahl deutlich reduziert wird. Zur Veranschaulichung zeigt **Figur 3** ein Raster von 3x3 Pupillenpunkten **73** innerhalb der Iris **74.** Für eine Beleuchtung mit einer Lichtschlitzblende 6 bzw. eines Lichtschlitzrasters **30** sind die Lichtschlitzbilder **22, 22'** und **22"** in der Pupille **12** dargestellt. Da die Pupille 12 auf das Detektor-Array 15 abgebildet wird, hat man beispielsweise bei Ray Tracing Messung in 10 x 10 Pupillenpunkten **73** am Detektorarray **15** nur mehr 1/10 der Detektoren **25** der insgesamt zur betreffenden x-Position des Pupillenpunkts (x, y) gehörenden Spalte des Detektorarrays **15** zur Verfügung. Die Feldtiefe wird damit auf 1/10 des maximal möglichen Wertes reduziert. Für eine CCD-Kamera mit etwa 2000 Detektoren ergibt sich somit eine Feldtiefe von rund 10 mm, was für die Vorderkammer in den meisten Fällen schon genügt. Bei 10 x 10 Messstrahlen durch die Pupille reduziert sich diese Feldtiefe jedoch auf etwa 1 mm, was kaum zur Korneadickenmessung reicht.

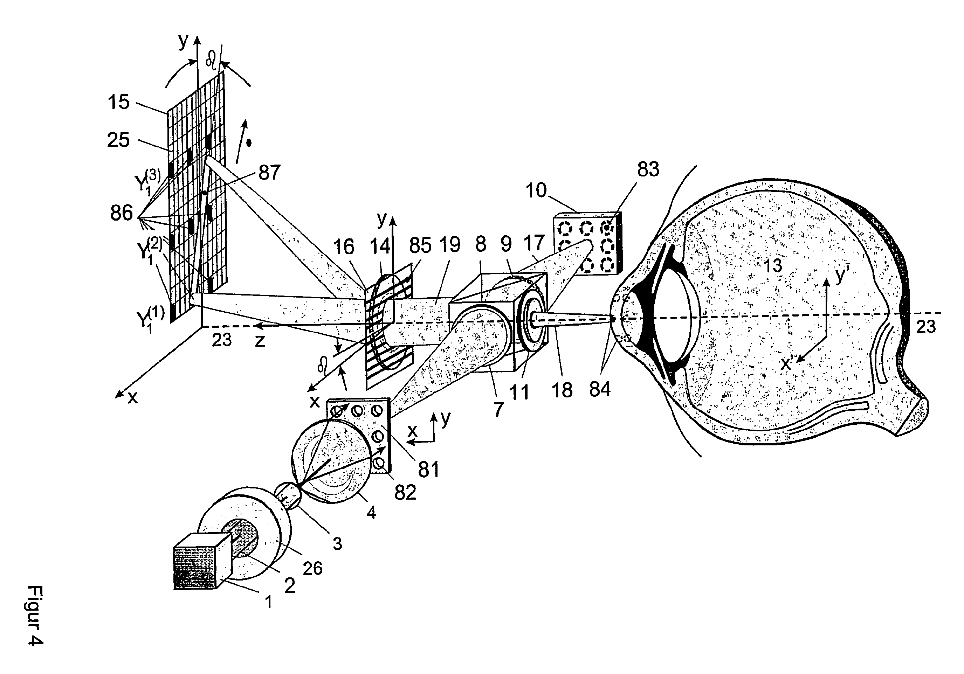

Eine weitere Ausgestaltungsvariante eines Kurzkohärenz-Interferometer für Ray Tracing ist in **Figur 4** dargestellt. Bei dieser Variante kann durch Verdrehen des Beugungsgitters um den Winkel β eine höhere Auflösung erreicht werden.

Nach **Figur 4** beleuchtet der aus der kurzkohärenten Beleuchtungsquelle **1** ausgehende und durch den Verschluss **26** hindurch tretende Lichtstrahl **2** mittels der Optiken **3** und **4** den Blendenraster **81** mit den Blendenöffnungen **82,** die hier beispielsweise in 3 Zeilen in x-Richtung und 3 Spalten in y-Richtung angeordnet sind.

Der Blendenraster **81** wird einerseits von den Optiken **7** und **9** durch den Strahlteiler **8** hindurch auf den Referenzspiegel **10** abgebildet und andererseits von der Optik **7** über die Teilerfläche des Strahlteilers **8** mittels der Optik **11** auf die Pupille **12** des Auges **13.** Die Blendenbilder 83 auf dem Referenzspiegel **10** und 84 auf dem Auge **13** werden von den Optiken **11, 9** und **14** durch das Beugungsgitter **16** auf das Detektorarray **15** abgebildet. Die Optik **14,** das Detektorarray **15** und das Beugungsgitter **16** bilden das Spektrometer, welches das Intensitätsspektrum des Lichtstrahls **19** spektral analysiert. Der Lichtstrahl **19** besteht aus den überlagerten Referenzstrahlen **17** und den Objektstrahlen **18** aller Blendenöffnungen **82.** Das Beugungsgitter **16** dispergiert die spektralen Komponenten des Lichtstrahles **19** in Richtung normal zu den Gitterlinien **85.**

Auch hier bilden die am Detektorarray **15** spaltenweise in Abhängigkeit von der Wellenlänge in y-Richtung gemessenen spektrometrischen Intensitäten die Ausgangsdaten für die Fouriertransformation zur Berechnung der Objektstruktur entlang Messstrahlen in den x'- und y'-Positionen am Auge **13.** In der Spektrometrie wird das Beugungsgitter **16** üblicherweise so orientiert, dass seine Gitterlinien **85** normal zur y-Richtung des (eindimensionalen) Detektorarrays **15** (parallel zur x-Richtung) orientiert sind, weil sonst die spektrale Auflösung verschlechtert wird. Die bisher beschriebenen Anordnungen basieren ebenfalls auf dieser Ausrichtung des Beugungsgitters **16.**

Die durch Überlagerung der Referenzstrahlen **17** und der Objektstrahlen **18** entstehenden Lichtstrahlen **19** erzeugen auf dem Detektorarray **15** Lichtschlitzbilder in den Positionen $Y_1^{(1)}$, $Y_1^{(2)}$, $Y_1^{(3)}$, $Y_2^{(1)}$, $Y_2^{(2)}$, $Y_2^{(3)}$ und $Y_3^{(1)}$, $Y_3^{(2)}$, $Y_3^{(3)}$. Für die spektrale Auflösung stehen hier nur die zwischen den Positionen $Y_1^{(1)}$ und $Y_1^{(3)}$ liegenden Detektoren **25** der betreffenden Spalte zur Verfügung. Die Detektoren **25** der anderen Spalten bleiben ungenutzt.

Durch Verdrehen des Beugungsgitters **16** um den azimutalen Winkel β erfolgt die wellenlängenabhängige Dispersion des Lichtbündels **19** nun als Spektrum **87** in eine um den azimutalen Winkel β zur y-Achse geneigte Richtung. Der für das Spektrum **87** zur Verfügung stehende Bereich auf dem Detektorarray **15** kann dadurch vergrößert werden. Das Beugungsgitter **16** wird dazu so gedreht, dass seine Gitterlinien **85** einen azimutalen Winkel β (in der x-y-Ebene) mit der x-Achse einschließen.

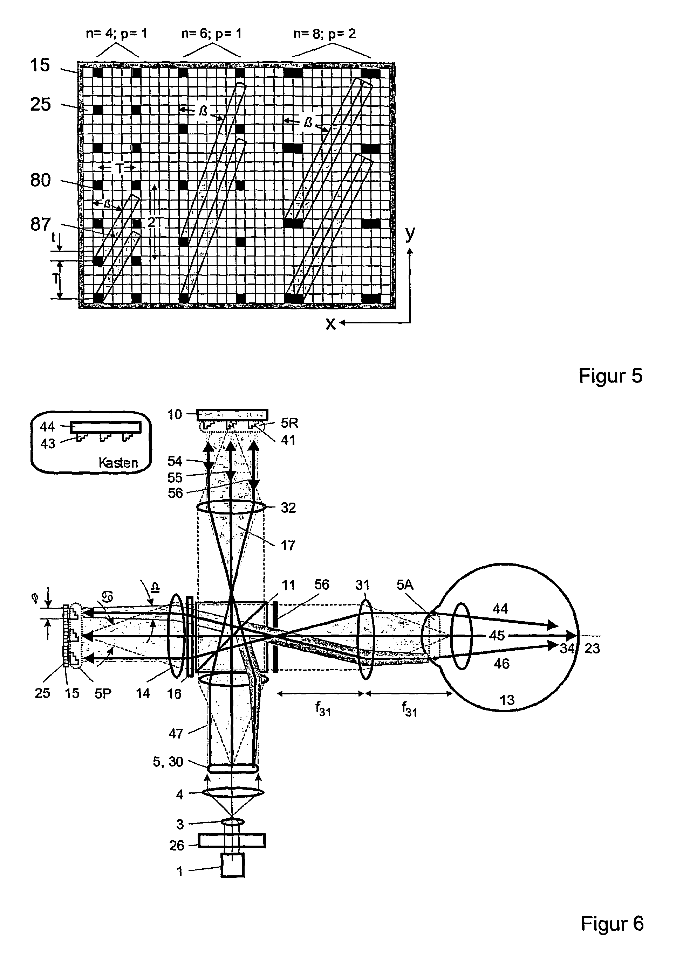

In **Figur 5** ist ein Detektorarray **15** mit verschiedenen Abbildungsmustern für die Lichtstrahlen **19** dargestellt. Das Detektorarray **15** besteht aus N mal N (mit beispielsweise N=2048) quadratischen Detektoren 25. In die schwarz markierten Detektoren **80** wird beispielsweise die kürzeste Wellenlänge des Spektrums **87** abgebildet.

Im linken Drittel der **Figur 5** entfallen auf eine Teilung T des Blendenrasters $n = 4$ Detektoren **25,** im mittleren Drittel $n = 6$. Die Gitterdispersion muss nun die anderen Wellenlängen so dispergieren, dass Spektren benachbarter Blendenpunkte nicht auf gleiche Detektoren **25** fallen. Wie man aus **Figur 5** ablesen kann, ist

$$\tan \beta = \frac{n}{n.n/2} = \frac{2}{n}.$$ Dreht man das Beugungsgitter **16** um den azimutalen Winkel β, so hat man näherungsweise $n = 2/\tan \beta$ Detektoren **25** zur Auflösung des Spektrums zur Verfügung und kann so die Feldtiefe entscheidend vergrößern.

Diese Bedingung gilt streng nur unter der Annahme, dass die empfindlichen Regionen der Detektoren **25** direkt aneinander grenzen, was bei CCD-Arrays weitgehend der Fall ist. Außerdem dürfen die Spektren **87** der einzelnen Blendenöffnungen **82** die Breite der Detektoren **25** nicht überschreiten. Ansonsten muss der Winkel β entsprechend vergrößert werden und die spektrale Auflösung wird etwas schlechter. Befinden sich zwischen den Detektoren **25** hingegen lichtunempfindliche Zonen, können die oben abgeschätzten Winkel β auch größer und damit die Auflösung weiter verbessert werden.

Entscheidend ist der azimutale Winkel β zwischen dem Messstrahlen- oder Blendenraster **81** und dem Beugungsgitter **16**. Das Detektorarray **15** kann davon unabhängig in verschiedene Richtung gedreht werden. Beispielsweise kann es nützlich sein, auch das Detektorarray **15** um den Winkel β zu drehen. Dann fallen die Spektren **87** der Messstrahlen auf die Detektoren **25** einer einzigen Array-Spalte, was den Ausleseprozess vereinfachen kann.

Die bisher beschriebenen Anordnungen basieren auf konventioneller Fourier-Domain OCT, die bekanntermaßen neben der gewünschten Objektstruktur umfangreiche Autokorrelationsterme liefert, wodurch die Abbildungstiefe der Fourier Domain OCT beschränkt wird.

Es ist bereits bekannt, dass Autokorrelationsterme vermieden werden können, wenn man mehrere Intensitätsspektren mit unterschiedlichen Phasen der Referenzstrahlung misst. Siehe dazu Kapitel "Optical Coherence Tomography in Medicine" im Band 4 der Reihe "Optical Sciences: International Trends in Optics and Photonics ICO IV" (erschienen 1999 im Springer Verlag, Berlin, Herausgeber: T. Asakura, Seiten 359-389). Hierzu sind mehrere spektrale Intensitätsmessungen auszuführen, wobei die Weglängen der Referenzwellen zwischen den einzelnen Messungen um Bruchteile einer Lichtwellenlänge zu verändert werden.

In der **Figur 6** ist der Strahlverlauf eines OCT-Gerätes dargestellt, welches auf der Phasenmultiplex Fourier-Domain Technik basiert.

Im Gegensatz zu den bisher beschriebenen Anordnungen wird hier ein Referenzspiegel **10** benutzt, der auf seiner Spiegelfläche ein stufenförmiges verspiegeltes Phasenprofil aufweist. Dieses besteht aus einer periodischen Anordnung von Phasentreppen **41** mit 2 oder mehr Stufen. Die Stufenhöhen sind dabei kleiner als eine Lichtwellenlänge.

Alternativ kann auch ein transparentes Phasenprofil vorhanden sein. Hierbei werden die Phasentreppen als transparente Schichten entsprechender Dicke auf eine transparente Platte aufgebracht, die in dem Strahlengang zwischen Strahlteiler **11** und Referenzspiegel **10** angeordnet wird.

Das Phasenprofil besteht aus periodisch angeordneten Treppen mit wenigen Stufen, die kleiner als □ sind. In der **Figur 6** besteht die Phasentreppe beispielsweise aus drei Stufen, die eine Stufenhöhe von □/8 aufweisen. Zu den 3 dargestellten Messstrahlen **44, 45** und **46** gehören die entsprechenden Referenzstrahlen **54, 55** und **56** mit zugehöriger Phasentreppe.

Die Mess- und Referenzstrahlen können auch Teil eines einzigen breiten Interferometer-Strahlenbündels sein. Während die Referenzstrahlen **54, 55** und **56** aufgrund der im Referenzstrahlengang vorliegenden großen Apertur (Aperturwinkel □) die Phasenstufen transversal aufgelöst auf das Detektorarray **15** abbilden, werden die Messstrahlen **44, 45** und **46** wegen der durch die Lochblende **56** begrenzten kleineren Apertur im Messstrahlengang (Aperturwinkel □) virtuell auf eine ganze Phasentreppe defokussiert. Dadurch können für jeden Messstrahl die zu den verschiedenen Referenzphasen gehörigen Intensitätswerte am Detektorarray **15** separat ausgelesen werden.

Die Lochblende **56** kann dabei gleichzeitig dazu benutzt werden, die Energiestromdichte des beleuchtenden Strahls auf den für das Auge **13** zulässigen Wert zu begrenzen.

Wenn je Messstrahl drei Phasenstufen vorliegen, wird der pro Phasenmessung verfügbare Lichtstrom um den Faktor drei reduziert. Man erleidet somit formal eine Reduktion des Signal-zu-Rausch Verhältnisses von 5dB und gewinnt einen Faktor drei in der Messzeit. Da man aber das Auge bei kürzerer Messzeit auch mit höherer Strahlleistung belasten kann, ist der Gewinn in der Messzeit gar nicht mit einem Verlust an Signal-zu-Rausch Verhältnis verbunden.

Außerdem ist hier keine Referenzspiegelbewegungen erforderlich und das Auslesen erfolgt nur einmal (One-Shot-Aufnahme), so dass sich Zeitvorteile ergeben, die deutlich größer sind als der Faktor drei.

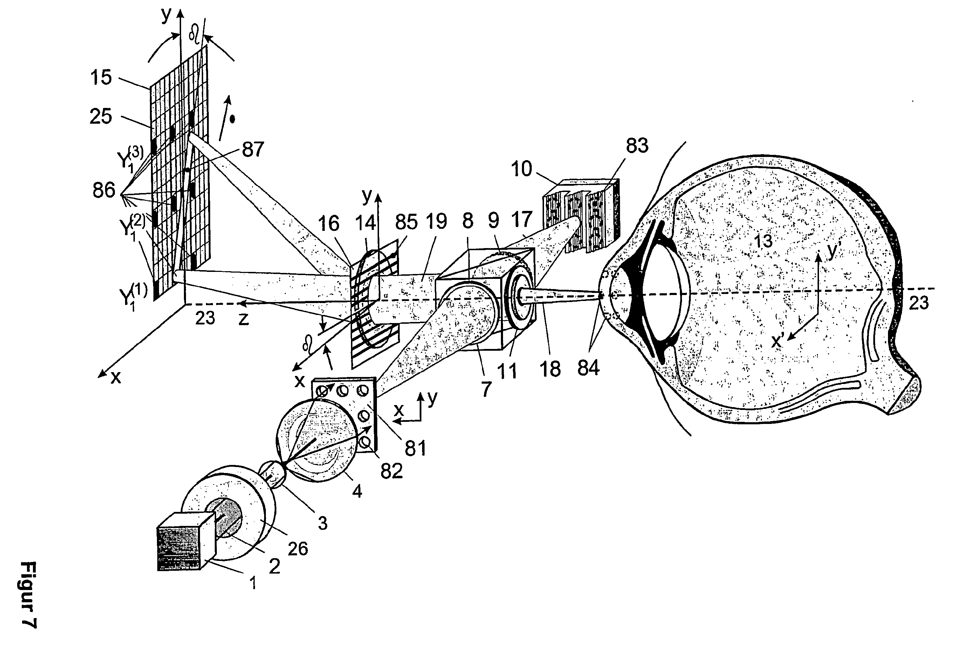

**Figur 7** zeigt den Strahlverlauf einer Anordnung für Phasenmultiplex Fourier OCT, bei welchem das Beugungsgitter um den Winkel β verdreht ist.

Bei dieser Lösung beleuchtet der aus der kurzkohärenten Beleuchtungsquelle **1** ausgehende und durch den Verschluss **26** hindurch tretende Lichtstrahl **2** mittels der Optiken **3** und **4** ein Blendenraster **81** mit den Blendenöffnungen **82,** die hier beispielsweise in 3 Zeilen und 3 Spalten symmetrisch angeordnet sind.

Der Blendenraster **81** wird einerseits von den Optiken **7** und **9** durch den Strahlteiler **8** hindurch auf den Referenzspiegel **10** mit dem Phasenprofil 83 abgebildet und andererseits von der Optik **7** über die Teilerfläche des Strahlteilers **8** mittels der Optik **11** auf die Pupille des Auges **13.** Die Blendenbilder **83** auf dem Referenzspiegel **10** und **84** auf dem Auge **13** werden von den Optiken **11, 9** und **14** durch das Beugungsgitter **16** auf das Detektorarray **15** abgebildet. Die Optik **14,** das Detektorarray **15** und das Beugungsgitter **16** bilden das Spektrometer, welches das Intensitätsspektrum des Lichtstrahls **19** spektral analysiert. Der Lichtstrahl **19** besteht aus den überlagerten Referenzstrahlen **17** und den Objektstrahlen **18** aller Blendenöffnungen **82.** Das Beugungsgitter **16** dispergiert die spektralen Komponenten des Lichtstrahles **19** in Richtung normal zu den Gitterlinien **85.**

Auch hier bilden die am Detektorarray **15** spaltenweise in Abhängigkeit von der Wellenlänge in y-Richtung gemessenen spektrometrischen Intensitäten die Ausgangsdaten für die Phasenmultiplex Fouriertransformation zur Berechnung der Objektstruktur entlang Messstrahlen in den x'- und y'-Positionen am Auge **13.**

Durch Verdrehen des Beugungsgitters **16** um den azimutalen Winkel β erfolgt die wellenlängenabhängige Dispersion des Lichtbündels **19** nun als Spektrum **87** in eine um den azimutalen Winkel β zur y-Achse geneigte Richtung. Der für das Spektrum **87** zur Verfügung stehende Bereich auf dem Detektorarray **16** kann so auch hier vergrößert werden.

Bei dem erfindungsgemäßen Fourier Domain OCT Ray Tracing am Auge wird die Pupille **12** des Auges **13** von einer kurzkohärenten Beleuchtungsquelle **1** in mehreren Punkten beleuchtet. Für die dabei verwendete Blendenanordnung sind mehrere Varianten denkbar. Zum einen kann eine Lochblende verwendet werden, die das Auge in verschiedenen Punkten nacheinander beleuchtet, oder es wird eine Schlitzblende verwendet die nur in einer Richtung über das Auge bewegt werden muss. Zum anderen ist es auch möglich Blendenraster zu verwenden, die über Blendenöffnungen oder Blendenschlitze verfügen und alle Punkte auf einmal beleuchten. Eine weitere Variante ist die Verwendung von sogenannten Linsenrastern.

Im folgenden wird auf die Verwendung einer bewegten Lichtblende näher eingegangen. Der Lichtschlitz kann zum einen beispielsweise mittels eines Schrittantriebes konsekutiv in die Messpositionen verschoben werden.

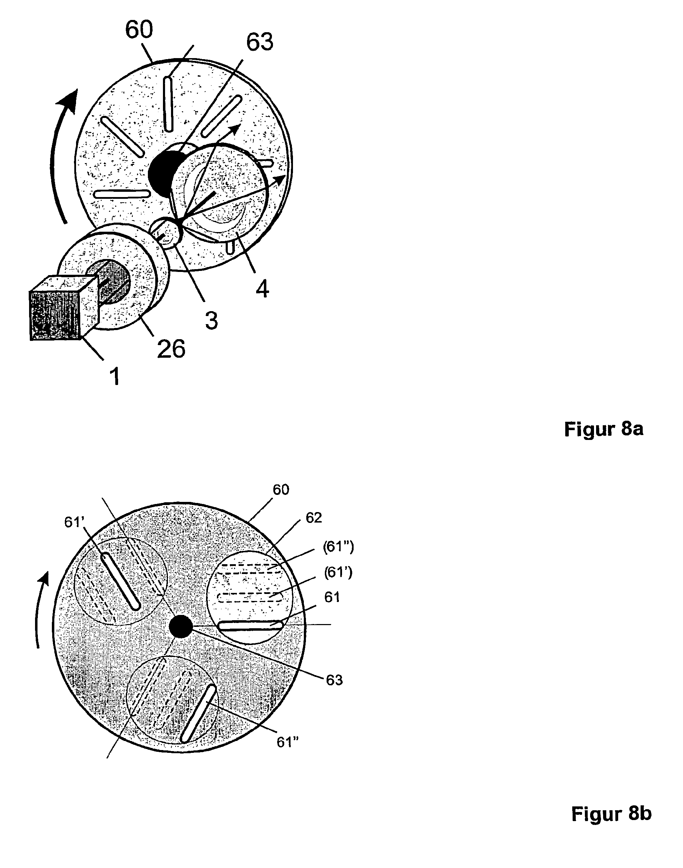

Zum anderen ist es aber auch möglich, eine rotierende Blendenanordnung zu benutzen. **Figur 8a** zeigt dazu die Anordnung von Schlitzblenden auf einer rotierenden Scheibe **60.** Wegen der orthogonalen Anordnung der Detektoren **25** in dem Detektorarray **15** darf auf dieser Scheibe jedoch nur eine einzige Lichtschlitzöffnung **61** genau in radialer Richtung orientiert sein, was durch die drei am Umfang der Scheibe **60** verteilten Lichtschlitzöffnungen **61, 61'** und **61"** dargestellt ist. Während einer Umdrehung der Scheibe **60** werden in den drei Lichtschlitzpositionen **61, 61'** und **61"** A-Scan Messungen in der abbildungsmäßig konjugierten Augenpupille **62** durchgeführt. Die Anzahl der auf der Scheibe **60** angeordneten Lichtschlitzöffnungen **61** ist dabei variabel.

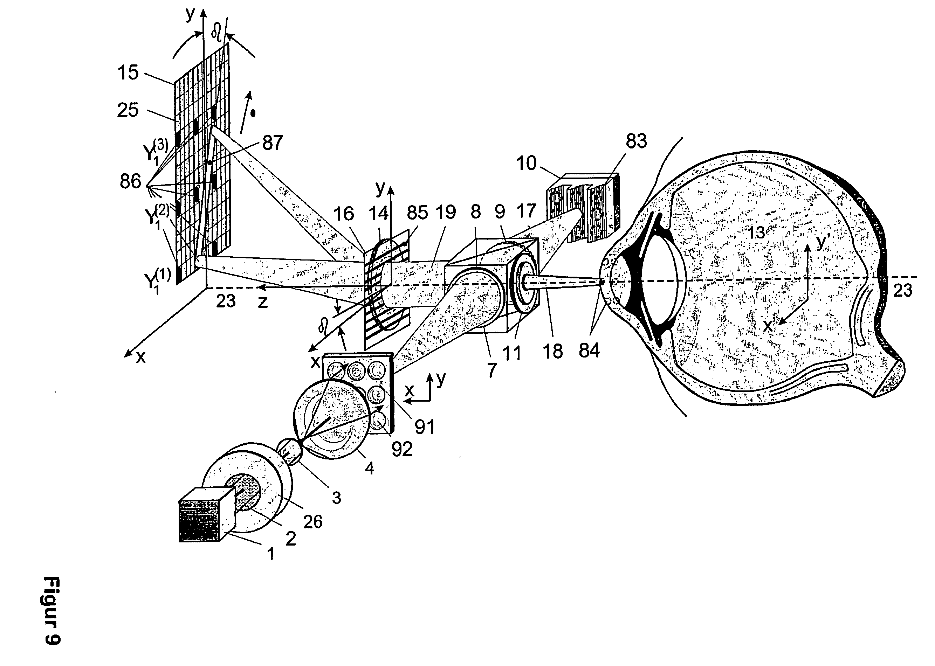

Schließlich kann man, wie in **Figur 9** dargestellt, anstelle des Blendenrasters **81** einen Linsenraster **91** benutzen. Dieser hat gegenüber dem Blendenraster **81** einen deutlich größeren Lichtleitwert. Der Linsenraster **91** wird ebenfalls durch den von der kurzkohärenten Beleuchtungsquelle **1** ausgehenden und durch den Verschluss **26** hindurch tretenden Lichtstrahl **2** mittels der Optiken **3** und **4** beleuchtet. Die Brennpunkte der Einzellinsen **92** des Linsenrasters **91** treten hier an die Stelle der Blendenöffnungen **82** beispielsweise der Anordnung nach **Figur 7.**

Bei den beschriebenen Fourier Domain OCT Verfahren erfolgt die Darstellung einer dreidimensionalen Struktur aller intraokulären brechenden und reflektierenden Grenzflächen und Oberflächen des Auges vorzugsweise mittels Spline- oder Polygonflächen.

Mit dem vorgeschlagenen Verfahren ist es möglich die Tiefenpositionen der Durchstoßpunkte und Reflexionspunkte der Messstrahlen in vielen Pupillenpunkten mit einer einzigen Aufnahme der Array-Kamera zu bestimmen. Dies kann durch erreicht werden, dass die Pupille mit einem Blendenraster beleuchtet wird und der Referenzspiegel einen periodischen Phasenraster enthält. Das Verfahren liefert bei einer große Objekttiefe eine hohe Bildqualität.

Außerdem kann das Fourier Domain OCT Ray Tracing am Auge wesentlich verbessert werden. Zum einen wird durch Verdrehen des Beugungsgitters um einen Winkel das Auflösungsvermögen wesentlich verbessert. Durch die Verwendung von Blenden- oder Linsenrastern ist es möglich die Messzeit durch "One-Shot"-Verfahren wesentlich zu verkürzen und dadurch Bewegungsartefakte zu vermeiden. Die vorliegende Lösung basiert auf den für Fourier-Domain OCT modifizierten physikalischen Grundlagen der spektralen Amplituden- und Phasenmessung. Durch einen Referenzstrahl mit räumlich periodischer Phasenrasterung entfällt das Verschieben des Referenzspiegels in verschiedene Positionen.

**Patentansprüche**

1. Fourier Domain OCT Ray Tracing am Auge, bei dem die Pupille von einer kurzkohärenten Beleuchtungsquelle (1) in mehreren Punkten beleuchtet und die in diesen Punkten von den Grenzflächen und Oberflächen des Auges (13) reflektierte Messstrahlung (18) mit einer Referenzstrahlung (17) überlagert wird, die Messdaten dieser sogenannten Stützpunkte am Ausgang des Interferometers spektral aufgespalten und zum gleichzeitigen Auslesen zweidimensionaler spektral-interferometrischer Daten und zweidimensionaler Ortsdaten am Ausgang des Interferometers auf ein zweidimensionales Detektorarray (15) abgebildet und an eine Steuereinheit weitergeleitet werden, die anhand dieser spektral-interferometrisch bestimmten Stützpunkte die Darstellung einer dreidimensionalen Struktur von intraokulären brechenden und/oder reflektierenden Grenzflächen und Oberflächen des Auges (13) ermittelt, bei dem zur Beleuchtung der Pupille (12) in mehreren Punkten eine bewegliche Blendenöffnung (5), ein Blendenraster (81) oder ein Linsenraster (91) verwendet wird.

2. Fourier Domain OCT Ray Tracing nach Anspruch 1, bei dem ein Beugungsgitter (16) zum spektralen Aufspalten der Messdaten am Ausgang des Interferometers vorgesehen ist, welches als Transmissions- oder Reflexionsgitter ausgeführt sein kann.

3. Fourier Domain OCT Ray Tracing nach Anspruch 2 bei dem durch die Einführung eines azimutalen Winkels β ≠ 0 zwischen dem aus dem Strahlteiler (8) austretenden Lichtstrahl (19) und Beugungsgitter (16) eine Erhöhung der Auflösung in der Fourier Domain OCT erreicht werden kann.

4. Fourier Domain OCT Ray Tracing nach Anspruch **3**, bei dem der zur Erhöhung der Auflösung in der Fourier Domain OCT eingeführte azimutale Winkels β zwischen Lichtstrahl (19) und Beugungsgitter (16), vorzugsweise

$$\tan \beta = \frac{n}{n.n/2} = \frac{2}{n}$$

beträgt, wobei $n$ der Anzahl der Detektoren (25) je Blendenraster-Teilung entspricht.

5. Fourier Domain OCT Ray Tracing nach Anspruch 1, bei dem der Referenzspiegel (10) zur Erzeugung einer Referenzstrahlung (17) mit räumlich periodischem Phasenprofil ein stufenförmiges Phasenprofil aufweist, deren Stufenhöhen kleiner als die Wellenlänge der kurzkohärenten Beleuchtungsquelle sind.

6. Fourier Domain OCT Ray Tracing nach Anspruch 5, bei dem das stufenförmige Phasenprofil des Referenzspiegels (10) eine, zwei oder mehrere Phasenstufen (41) ausweisen und als verspiegeltes oder transparentes Oberflächenprofil ausgeführt sein kann.

7. Fourier Domain OCT Ray Tracing nach Anspruch 4, bei dem der zur Erhöhung der Auflösung in der Fourier Domain OCT eingeführte azimutale Winkels β zwischen Lichtstrahl (19) und Beugungsgitter (16), vorzugsweise

$$\tan \beta = p \frac{2}{n}$$

beträgt, wobei $n$ der Anzahl der Detektoren (25) je Blendenraster-Teilung und $p$ der Anzahl der Messdatensätze mit verschiedenen Referenzphasen entspricht.

8. Fourier Domain OCT Ray Tracing nach mindestens einem der vorgenannten Ansprüche, bei dem das zweidimensionale Detektorarray (15) vorzugsweise ebenfalls um den azimutalen Winkels β verdreht wird, so dass die Spektren der Messstrahlen in Richtung der Detektorspalten auf das Detektorarray (25) fallen.

9. Fourier Domain OCT Ray Tracing nach mindestens einem der vorgenannten Ansprüche, bei dem die Darstellung einer dreidimensionalen Struktur aller intraokulären brechenden und reflektierenden Grenzflächen und Oberflächen des Auges (13) mittels Spline- oder Polygonflächen erfolgt.

**Claims**

1. Fourier domain OCT ray tracing on the eye, in the case of which the pupil is illuminated at a plurality of points by a short-coherence illumination source (1), and a reference radiation (17) is superimposed on the measurement radiation (18) reflected at these points by the interfaces and surfaces of the eye (13), the measured data of these so-called interpolation points are spectrally split at the output of the interferometer and imaged on a two-dimensional detector array (15) for the purpose of simultaneously reading out two-dimensional spectral interferometric data and two-dimensional spatial data at the output of the interferometer, and are passed on to a control unit which uses these interpolation points determined by spectral interferometry to determine the representation of a three-dimensional structure of refracting and/or reflecting intraocular interfaces and surfaces of the eye (13), in which a moving diaphragm aperture (5), a diaphragm grid (81), or a lens grid (91) is used to illuminate the pupil (12) at a plurality of points.

2. Fourier domain OCT ray tracing according to Claim 1, in which there is provided at the output of the interferometer for the purpose of spectral splitting of the measured data a diffraction grating (16) which can be designed as a transmission or reflection grating.

3. Fourier domain OCT ray tracing according to Claim 2, in which an increase in the resolution can be achieved in the Fourier domain OCT by introducing an azimuth angle $\beta \neq 0$ between the light beam (19) exiting from the beam splitter (8) and the diffraction grating (16).

4. Fourier domain OCT ray tracing according to Claim 3, in which the azimuth angle $\beta$ between the light beam (19) and diffraction grating (16) for the purpose of increasing the resolution in the Fourier domain OCT is preferably

$$\tan \beta = \frac{n}{n.n/2} = \frac{2}{n}$$

$n$ corresponding to the number of the detectors (25) per diaphragm grid division.

5. Fourier domain OCT ray tracing according to Claim 1, in which the reference mirror (10) for generating a reference beam (17) with a spatially periodic phase profile has a step-shaped phase profile whose step heights are smaller than the wavelength of the short-coherence illumination source.

6. Fourier domain OCT ray tracing according to Claim 5, in which the step-shaped phase profile of the reference mirror (2) can have one, two or more phase steps (41) and be designed as reflective or transparent surface profile.

7. Fourier domain OCT ray tracing according to Claim 4, in which the azimuth angle $\beta$ introduced between the light beam (19) and diffraction grating (16) for the purpose of increasing the resolution in the Fourier domain OCT is preferably

$$\tan \beta = p \frac{2}{n}$$

$n$ being the number of the detectors (25) per diaphragm grid division, and p corresponding to the number of the measured data records with different reference phases.

8. Fourier domain OCT ray tracing according to at least one of the abovementioned claims, in which the two-dimensional detector array (15) is preferably likewise rotated by the azimuth angle $\beta$ such that the spectra of the measurement beams impinge on the detector array (25) in the direction of the detector columns.

9. Fourier domain OCT ray tracing according to at least one of the abovementioned claims, in which the representation of a three-dimensional structure of all the refracting and reflecting intraocular interfaces and surfaces of the eye (13) is performed by means of spline surfaces or polygonal surfaces.

**Revendications**

1. Lancer de rayon OCT dans le domaine de Fourier sur l'oeil, dans lequel la pupille est illuminée en plusieurs points par une source d'illumination (1) à courte cohérence et dont le rayonnement de mesure (18) réfléchi en ces points par les surfaces d'interfaces et les surfaces externes de l'oeil (13), est superposé à un rayonnement de référence (17), les données de mesure de ces dits points d'appui sont séparées du point de vue spectral à la sortie de l'interféromètre et sont reproduites sur une matrice de détection bidimensionnelle (15) pour la lecture simultanée des données bidimensionnelles d'interférométrie spectrale et des données bidimensionnelles de localisation à la sortie de l'interféromètre, et sont retransmises vers une unité de commande qui, à l'aide de ces points d'appui définis par interférométrie spectrale, détermine la représentation d'une structure tridimensionnelle des surfaces d'interfaces intraoculaires de réfraction et/ou de réflexion et des surfaces externes de l'oeil (13), dans lequel on utilise un diaphragme mobile (5), un balayage par diaphragme (81) ou un balayage par lentille (91) pour l'illumination de la pupille (12) en plusieurs points.

2. Lancer de rayon OCT dans le domaine de Fourier selon la revendication 1, dans lequel une grille de diffraction (16) qui peut être réalisée sous la forme d'une grille de transmission ou de réflexion, est prévue pour la séparation spectrale des données de mesure à la sortie de l'interféromètre.

3. Lancer de rayon OCT dans le domaine de Fourier selon la revendication 2, dans lequel l'introduction d'un angle azimutal $\beta \neq 0$ entre le faisceau lumineux (19) sortant du séparateur de faisceau (8) et la grille de diffraction (16) permet d'obtenir une augmentation de la résolution dans le domaine OCT de Fourier.

4. Lancer de rayon OCT dans le domaine de Fourier selon la revendication 3, dans lequel l'introduction de l'angle azimutal $\beta$ entre le faisceau lumineux (19) et la grille de diffraction (16) pour l'augmentation de la résolution dans le domaine OCT de Fourier vaut de préférence

$$\tan \beta = \frac{n}{n.n/2} = \frac{2}{n}$$

où $n$ correspond au nombre de détecteurs (25) par séparation de balayage du diaphragme.

**5.** Lancer de rayon OCT dans le domaine de Fourier selon la revendication 1, dans lequel le miroir de référence (10) destiné à la génération d'un rayonnement de référence (17) ayant un profil de phases périodique dans l'espace, présente une profil de phases en paliers dont les hauteurs des paliers sont inférieures à la longueur d'onde de la source d'illumination à courte cohérence.

**6.** Lancer de rayon OCT dans le domaine de Fourier selon la revendication 5, dans lequel le profil de phases en paliers du miroir de référence (10) présente un, deux ou plusieurs paliers de phases (41) et pouvant être réalisé avec un profil de surface réfléchissant ou transparent.

**7.** Lancer de rayon OCT dans le domaine de Fourier selon la revendication 4, dans lequel l'introduction de l'angle azimutal $\beta$ entre le faisceau lumineux (19) et la grille de diffraction (16) pour l'augmentation de la résolution dans le domaine OCT de Fourier vaut de préférence

$$\tan \beta = p \, \frac{2}{n}$$

où $n$ correspond au nombre de détecteurs (25) par séparation de balayage du diaphragme et où $p$ correspond au nombre de séries de données de mesures avec des phases de référence différentes.

**8.** Lancer de rayon OCT dans le domaine de Fourier selon une au moins des revendications énoncées précédemment, dans lequel la matrice de détection bidimensionnelle (15) est de préférence également tournée d'un angle azimutal $\beta$ de telle sorte que les spectres des rayonnements de mesure en direction des colonnes du détecteur tombent sur la matrice de détecteurs (25).

**9.** Lancer de rayon OCT dans le domaine de Fourier selon une au moins des revendications énoncées précédemment, dans lequel la représentation d'une structure tridimensionnelle de toutes les surfaces d'interfaces intraoculaires de réfraction et de réflexion et des surfaces externes de l'oeil (13), a lieu au moyen de surfaces de spline et de surfaces polygonales.

Figur 1

Figur 2

Figur 3

Figur 4

Figur 5

Figur 6

Figur 7

**Figur 8a**

**Figur 8b**

**Figur 9**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4309056 A1
- US 6268921 B1

- US 6377349 B1

**In der Beschreibung aufgeführte Nicht-Patentliteratur**

- Aberrations and relative efficiency of light pencils in the living human eye. *Optometry-and-Vision-Science,* 1997, vol. 74 (7), 540-547
- **Navarro ; Moreno-Barriuso.** Laser ray-tracing method for optical testing. *Optics Letters,* 1999, vol. 24 (14), 951-953
- **Bouma, B. E. ; Tearney, G. J.** Handbook of Optical Coherence Tomography. Marcel Dekker Verlag, 2002
- **A. F. Fercher ; Mitarbeitern.** Measurement of optical distances by optical spectrum modulation. *Proc. SPIE,* 1993, vol. 2083, 263-267
- **Bellingham, W. A.** In Vivo Optical Coherence Tomography in Ophthalmology. *SPIE.,* 1993, ISBN 0-8194-1379-8, 355-370

- **A. F. Fercher ; Mitarbeitern.** Measurement of Intraocular Distances by Backscattering Spectral Interferometry. *Opt. Commun.,* 1995, vol. 117, 43-48
- Coherence RADAR'' and ''spectral RADAR''-New tools for dermatological diagnosis. *J. Biomed. Opt.,* 1998, vol. 3 (1), 21-31
- **A. G. Podoleanu ; J. A. Rogers ; D. A. Jackson ; S. Dunne.** Three dimensional OCT images from retina and skin. *Opt. Express,* 2000, vol. 7, 292-298
- **S. Radhakrishnan.** Real time optical coherence tomography of the anterior segment using hand-held and slit-lamp adapted systems. *Proc. SPIE,* 2002, vol. 4619, 227-229
- Optical Sciences: International Trends in Optics and Photonics ICO IV. Springer Verlag, 1999, 359-389